# EUROPEAN PATENT APPLICATION

(11) **EP 4 193 983 A1**
(43) Date of publication of application: **14.06.2023**
(21) Application number: 21856072.0
(22) Date of filing: 15.07.2021
(51) Int. Cl.: A61H 9/00, A61N 1/40, A61N 1/06, A61F 7/00, A61N 5/06, A61H 7/00

(54) **HANDPIECE CAPABLE OF ATTACHING/DETACHING VACUUM CAP AND ADJUSTING ANGLE OF VACUUM CAP**

(30) Priority: 10.08.2020 KR 20200100073; 10.08.2020 KR 20200120654
(71) Applicant: Park, Won Hee, Seoul 06715 (KR)
(72) Inventor: Park, Won Hee, Seoul 06715 (KR)
(74) Representative: Viering, Jentschura & Partner mbB Patent- und Rechtsanwälte
(86) International application number: PCT/KR2021/009088
(87) International publication number: WO 2022/035066

(57) **Abstract**

Provided in the present invention is a handpiece (300) for a treatment device with improved stability and hygiene function by being configured so as to freely adjust the angle of a vacuum cap depending on a treatment site and to attach/detach the vacuum cap to/from the handpiece (300). The handpiece (300) capable of attaching/detaching a vacuum cap and adjusting an angle of the vacuum cap comprises: a vacuum cap (310) having an open lower surface and a hollow part formed therein; a body part (410) configured to attach/detach the vacuum cap (310) thereto/therefrom; a vacuum device (350) for providing a reference negative pressure to the vacuum cap so that a target site of the skin can be adhered toward the hollow part of the vacuum cap; a reference negative pressure variable device (360) for varying the reference negative pressure in a pulse form under the condition in which the hollow part of the vacuum cap (310) maintains the negative pressure; RF electrodes (320, 321) configured to be exposed to the inside wall of the vacuum cap (310) so that, when the target site of the skin has been adhered toward the hollow part of the vacuum cap (310), the skin adhered toward the hollow part comes into contact with the inside of the hollow part of the vacuum cap (310); and a control unit (380) for controlling driving of the vacuum device (350), the reference negative pressure variable device (360), and the RF electrodes (320, 321). The driving of the reference negative pressure variable device (360) and the RF electrodes (320, 321) for treating the target site of the skin is controlled so as to select any one of single driving, simultaneous driving, and alternating driving. The attachment/detachment of the vacuum cap (310) to/from the body part (410) is made by simultaneous coupling or simultaneous separation of a vacuum cap connection unit (430) that consists of a vacuum tube coupler (432) configured at an end of the body part (410), power supply connectors (433, 434), and a clip (435).

## Description

### [Technical Field]

The present invention relates to a handpiece capable of attaching/detaching a vacuum cap and adjusting an angle of the vacuum cap, and more particularly, to a handpiece for a treatment device, capable of attaching/detaching a vacuum cap and adjusting an angle of the vacuum cap, in which safety, convenience of a treatment, and hygiene function are improved so that skin is effectively treated, or subcutaneous fat tissue is effectively decomposed and removed.

### [Background Art]

Korean Patent Registration No. 10-1055334 has been known as one example of a general treatment device using a vacuum cap according to the related art.

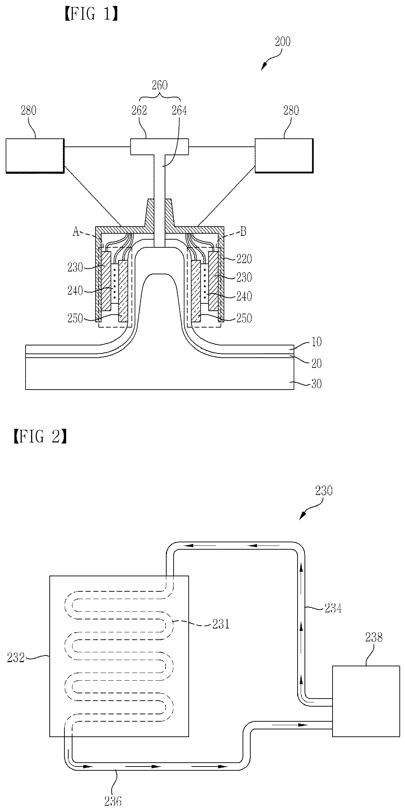

A treatment device 200 using a vacuum cap disclosed in Korean Patent Registration No. 10-1055334 will be described with reference to FIGS. 1 and 2.

As shown in FIG. 1, the treatment device 200 using the vacuum cap according to the related art may include a vacuum cap 220, a cooling member 230, a thermoelectric element 240, an RF high-frequency electrode 250, and a vacuum suction unit 260.

The vacuum cap 220 may have a socket shape formed therein with an empty space. The cooling member 230 may be mounted on each of both inner walls of the vacuum cap 220 facing each other.

As shown in FIG. 2, the cooling member 230 may include a cooling jacket 232, a coolant supply pipe 234, a coolant discharge pipe 236, and a chiller 238. The cooling jacket 232 may be provided therein with a coolant circulation pipe 231 through which a coolant circulates. In this case, the cooling jacket 232 may be attached to one surface of the thermoelectric element 240 to cool the thermoelectric element by the coolant circulating through the coolant circulation pipe 231 in order to cool down one side surface of the thermoelectric element, which has a temperature increased as much as a temperature of an opposite side surface of the thermoelectric element cooled by the Peltier effect, and the cooling member 230 corresponding to the cooled opposite side surface may cool a target site.

The coolant supply pipe 234 may supply the coolant to the coolant circulation pipe 231 provided inside the cooling jacket 232. The coolant supply pipe 234 may have one end connected to the cooling jacket 232 and an opposite end connected to the chiller 238 for circulating the coolant.

In other words, as shown in FIG. 1, since the thermoelectric element 240 has both surfaces attached to the cooling member 230 and the RF high-frequency electrode 250 while being interposed between the cooling member 230 and the RF high-frequency electrode 250, the cooling member 230 may serve as a cooling plate for the RF high-frequency electrode 250.

The RF high-frequency electrode 250 may be connected to a control unit 280 to generate an RF high-frequency wave.

Meanwhile, the vacuum suction unit 260 communicating with an inner ceiling of the vacuum cap 220 may include a vacuum generation unit 262 for generating a vacuum pressure and a vacuum hole 264 for receiving the vacuum pressure from the vacuum generation unit 262, so that the target site that requires a treatment may be adsorbed to the RF high-frequency electrode 250 by the vacuum pressure.

In addition, US Patent Registration No. US9, 168, 096 has been known as another example of a treatment device using a vacuum cap according to the related art.

A treatment device using a vacuum cap disclosed in US Patent Registration No. US9,168,096 will be described with reference to FIGS. 3 and 4.

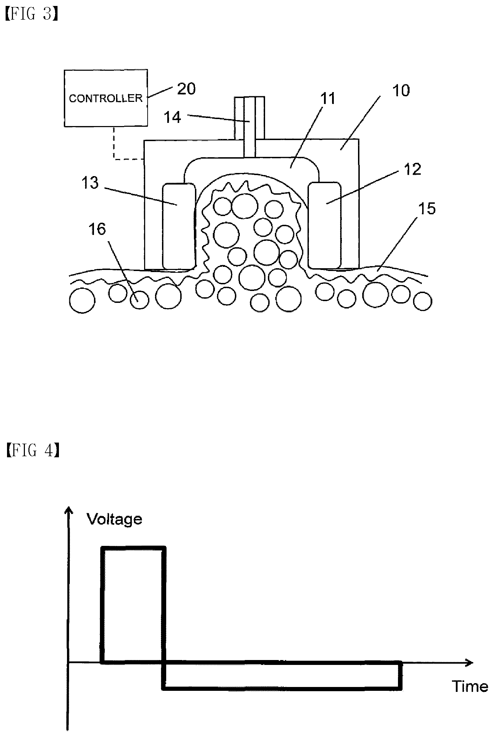

As shown in FIG. 3, the treatment device 100 using the vacuum cap according to the related art may include: a vacuum cap 10 formed therein with a hollow part 11, and having a cooling function; RF electrodes 12 and 13 formed on inner wall surfaces of the vacuum cap, respectively; a suction port 14 for forming a negative pressure in the hollow part; and a control unit 20 for controlling the negative pressure through the RF electrodes 12 and 13 and the suction port 14.

When the negative pressure is applied to the hollow part 11 after skin 15 that requires a treatment is attached to a bottom surface of the vacuum cap 10, the skin 15 and subcutaneous fat 16 may be sucked toward the hollow part so that the skin 15 may make contact with the RF electrodes 12 and 13.

In the above state, an alternating current having a waveform as shown in FIG. 4 may be applied through the RF electrodes 12 and 13 to decompose the subcutaneous fat so that obesity may be treated.

According to the treatment device 100 or 200 using the vacuum cap of the related art, which are configured as described above, a treatment may be performed by emitting RF waves to a selected site in which the skin and the subcutaneous fat are sucked into the hollow part of the vacuum cap 10 or 220 by vacuum, so that fat decomposition efficiency may be improved, or the treatment may be facilitated. However, there are limitations to repeatedly performing relaxation and contraction on fat tissue during a treatment process to facilitate decomposition of the fat tissue by physically stimulating the skin and the subcutaneous fat at a treatment site.

In addition, since one vacuum cap is used on skin of multiple people, it may be vulnerable to bacterial transmission or hygiene, and safety of attachment of the vacuum cap or fatigue of a wrist of an operator who grips a handpiece may be increased depending on a treatment site.

### [Disclosure]

### [Technical Problem]

To solve the problems described above, one object of the present invention is to provide a handpiece for a treatment device, in which an angle of a vacuum cap may be freely adjusted depending on a treatment site, and the vacuum cap may be attached/detached to/from the handpiece, so that stability, convenience of a treatment, and a hygiene function may be improved.

Another object of the present invention is to provide a handpiece for a treatment device, in which a function of forcibly and repeatedly performing relaxation and contraction of skin within a vacuum cap in a vertical direction of the vacuum cap may be configured so that a decomposition effect of subcutaneous fat tissue in a treatment site may be increased.

Still another object of the present invention is to provide a reference negative pressure variable device capable of selectively applying a pulsed fluctuating negative pressure for changing a reference negative pressure by a predetermined amplitude in a predetermined cycle for a predetermined time so that relaxation and contraction may be repeatedly performed on skin within a vacuum cap in a direction that is approximately perpendicular to a center line direction of the vacuum cap.

### [Technical Solution]

To achieve the objects described above, according to the present invention, a handpiece capable of attaching/detaching a vacuum cap and adjusting an angle of the vacuum cap includes:
a vacuum cap having an open bottom surface, and formed therein with a hollow part;
a first body part to/from which the vacuum cap is attached/detached;
a second body part coupled to the first body part with an angle adjustment device interposed between the first body part and the second body part so that an angle between the first body part and the second body part is adjusted; and
a vacuum device for providing a reference negative pressure to the vacuum cap so that a target site of skin is adsorbed toward the hollow part of the vacuum cap.

In addition, the angle adjustment device may be configured as a joint member or a rotation shaft for coupling the first body part to the second body part.

In addition, the handpiece may further include: the reference negative pressure variable device for varying the reference negative pressure in a pulse form under a condition in which the hollow part of the vacuum cap maintains a negative pressure;
a treatment unit for treating the target site of the skin; and
a control unit for controlling driving of the vacuum device, the reference negative pressure variable device, the treatment unit.

In addition, the first body part may include: a vacuum tube connected to the vacuum device and the reference negative pressure variable device; a vacuum tube coupler coupled to an end of the vacuum tube so as to be detachably coupled to the vacuum cap; and a power supply connector for driving the treatment unit.

In addition, a period of a reference negative pressure variable pulse generated by the reference negative pressure variable device may correspond to 0.5 to 50 times per second.

In addition, the treatment unit may be one selected from a treatment unit using electromagnetic waves, a treatment unit using cold and warm heat, and a treatment unit using light rays.

In addition, the treatment unit using the electromagnetic waves may include an RF electrode exposed toward the hollow part of the vacuum cap, and may be configured to allow a portion of the target site of the skin to make contact with the RF electrode when the target site of the skin is adsorbed toward the hollow part of the vacuum cap.

In addition, the driving of the reference negative pressure variable device and the treatment unit for treating the target site of the skin may be performed by selecting one of single driving, simultaneous driving, and alternating driving.

### [Advantageous Effects]

According to the present invention, a handpiece (300) capable of attaching/detaching a vacuum cap and adjusting an angle of the vacuum cap may include:
a vacuum cap (310) having an open bottom surface, and formed therein with a hollow part (315);
a first body part (410) to/from which the vacuum cap is attached/detached;
a second body part (420) coupled to the first body part with an angle adjustment device (415) interposed between the first body part and the second body part so that an angle between the first body part and the second body part is adjusted;
a vacuum device (350) for providing a reference negative pressure to the vacuum cap so that a target site of skin is adsorbed toward the hollow part (315) of the vacuum cap;
a reference negative pressure variable device (360) for varying the reference negative pressure in a pulse form under a condition in which the hollow part (315) of the vacuum cap maintains a negative pressure;
a treatment unit (RF electrodes (320, 321)) for treating the target site of the skin; and
a control unit (380) for controlling driving of the vacuum device, the reference negative pressure variable device, and the treatment unit, so that angle adjustment of the vacuum cap can be freely performed depending on a treatment site so as to effectively respond to a curved body site and reduce fatigue of an operator.

In addition, since the vacuum cap (310) may be attached/detached for one-time use, it may be unnecessary to sterilize the vacuum cap every time the vacuum cap is used, so that stability, convenience of a treatment, and hygiene can be ensured.

In addition, since relaxation/contraction is performed on a fat layer of the skin within the vacuum cap at a sufficiently large relaxation/contraction interval as compared with a treatment device having a simple vibration or massage function according to the related art, vertical relaxation between fat tissue in a treatment site can be promoted, so that a decomposition effect of subcutaneous fat tissue can be significantly improved.

### [Description of Drawings]

FIG. 1 is a sectional view for describing a treatment device using a vacuum cap according to the related art.
FIG. 2 is a view showing a cooling member applied to the treatment device using the vacuum cap of FIG. 1.
FIG. 3 is a sectional view for describing another example of a treatment device using a vacuum cap according to the related art.
FIG. 4 is a view showing an AC voltage waveform of one cycle for an RF electrode applied to the treatment device using the vacuum cap of FIG. 3.
FIG. 5 is a stereoscopic view schematically showing a handpiece according to the present invention.
FIG. 6 is a sectional view schematically showing the handpiece according to the present invention.
FIG. 7 is a sectional view schematically showing the handpiece in a state in which a vacuum cap is removed according to the present invention.
FIG. 8 is a stereoscopic view schematically showing the handpiece in which an angle is adjusted according to the present invention.
FIG. 9 is a view showing a state in which a target site of skin is adsorbed to the vacuum cap of FIG. 6.
FIG. 10 is a view illustrating a reference negative pressure variable pulse according to the present invention.
FIG. 11 is a view schematically showing a fat cell in the target site of the skin relaxed and disturbed by driving of a reference negative pressure variable device according to the present invention.
FIG. 12 is a flowchart for describing an operation of the handpiece capable of attaching/detaching the vacuum cap and adjusting the angle of the vacuum cap according to the present invention.

### [Best Mode]

A handpiece includes: a vacuum cap 310 having an open bottom surface open, and formed therein with a hollow part;
a body part 410 and 420 to/from which the vacuum cap is attached/detached;
a vacuum device 350 for providing a reference negative pressure to the vacuum cap so that a target site of skin is adsorbed toward the hollow part of the vacuum cap;
a reference negative pressure variable device 360 for varying the reference negative pressure in a pulse form under a condition in which the hollow part of the vacuum cap maintains a negative pressure;
an RF electrode 320 and 321 exposed to an inside wall of the vacuum cap so that the adsorbed skin makes contact with an inside of the hollow part of the vacuum cap when the target site of the skin is adsorbed toward the hollow part of the vacuum cap; and
a control unit 380 for controlling driving of the vacuum device, the reference negative pressure variable device, and the RF electrode.

The driving of the reference negative pressure variable device and the RF electrode for treating the target site of the skin is controlled by selecting one of single driving, simultaneous driving, and alternating driving.

The attachment/detachment of the vacuum cap to/from the body part is performed by simultaneous coupling and simultaneous separation of a vacuum cap connection unit 430 including a vacuum tube coupler 432, a power supply connector 433 and 434, and a clip 435, which are provided at an end of the body part.

In addition, the body part includes: a first body part 410 to/from which the vacuum cap is attached/detached; and a second body part 420 coupled to the first body part with an angle adjustment device, which is configured as a joint member or a rotation shaft, interposed between the first body part and the second body part so that a user bends the second body part to adjust an angle between the first body part and the second body part, and the vacuum cap connection unit 430 is provided at an end of the first body part 410.

In addition, the reference negative pressure variable device 360 is configured to vary the reference negative pressure in the pulse form having a period corresponding to 0.5 to 50 times per second by a pressure variable valve 361 under the condition in which the hollow part of the vacuum cap 310 maintains the negative pressure, and the vacuum device 350 is configured to automatically operate to maintain the reference negative pressure when the reference negative pressure within the hollow part of the vacuum cap 310 is out of a set range due to an adhesion failure occurring in a contact portion between the vacuum cap 310 and the skin.

### [Mode for Invention]

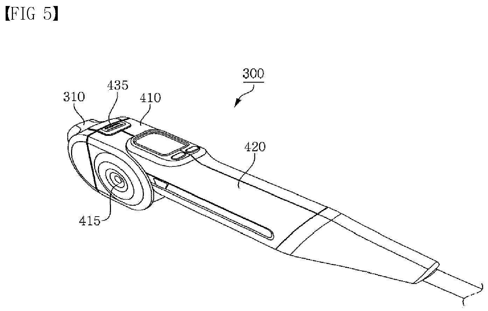

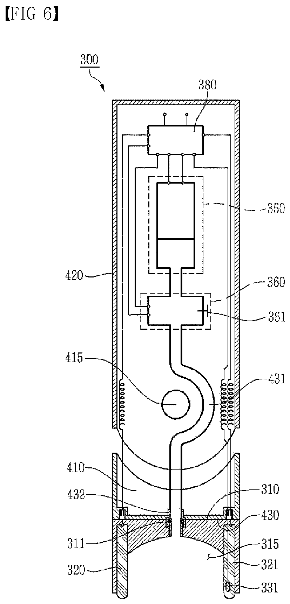

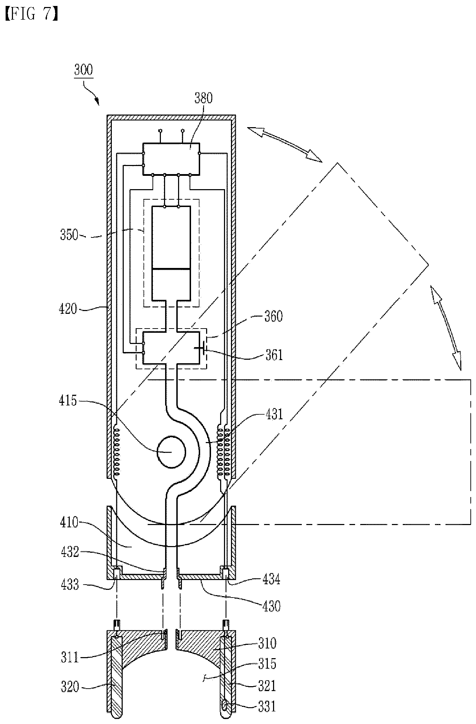

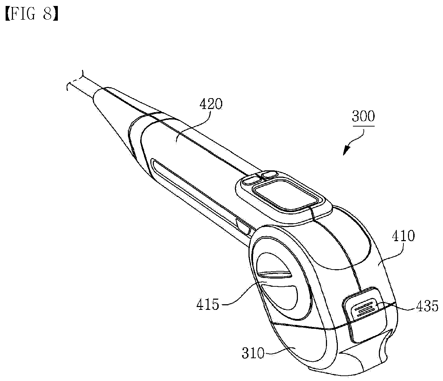

Hereinafter, the technical configuration and operations of a handpiece 300 capable of attaching/detaching a vacuum cap and adjusting an angle of the vacuum cap according to the present invention will be described in detail with reference to FIGS. 5 to 12.

As shown in FIGS. 5 to 8, according to the present invention, the handpiece 300 capable of attaching/detaching the vacuum cap and adjusting the angle of the vacuum cap may include: a first body part 410; a second body part 420 functioning as a handle; and a vacuum cap 310 having an open bottom surface, and formed therein with a hollow part.

An angle adjustment device may be interposed between the first body part 410 and the second body part 420 functioning as a handle so that an angle between the first body part 410 and the second body part 420 may be adjusted.

The angle adjustment device may be configured as a rotation shaft 415 for coupling one ends of the first body part 410 and the second body part 420 to each other, or may be configured as a joint member instead of the rotation shaft so that the second body part 420 may be bent in any direction with respect to the first body part 410.

In addition, a vacuum cap connection unit 430 for detachably fixing the vacuum cap 310 may be provided at an opposite end of the first body part 410.

The vacuum cap connection unit 430 may include: a vacuum tube 431 connected to the vacuum device 350 and the reference negative pressure variable device 360; a vacuum tube coupler 432 coupled to an end of the vacuum tube so as to be detachably coupled to the vacuum cap 310; power supply connectors 433 and 434 for driving a treatment unit such as RF electrodes 320 and 321; and a clip 435 for attaching/detaching the vacuum cap 310.

The vacuum tube 431 connected to the vacuum device 350 and the reference negative pressure variable device 360 may be preferably formed of a flexible material to prevent damage when the angle is adjusted by bending the second body part 420 from the first body part 410, and, if necessary, a portion (a region in which the angle adjustment device is located) of the vacuum tube 431 may be configured as a curved or spiral tube so as to have flexibility.

In addition, a portion of a power cable connected to each of the power supply connectors 433 and 434 may have a spiral shape so as to have flexibility when the angle is adjusted.

As described above, the angle between the first body part 410 and the second body part 420 functioning as a handle may be adjusted, and the vacuum cap 310 may be attached/detached to/from the first body part 410, so that the vacuum cap 310 may be effectively fixed to a curved body site, and fatigue of an operator may be reduced by arbitrarily and easily adjusting the angle of the second body part 420 constituting a handle part of the handpiece.

In addition, the vacuum cap 310 may be provided with the RF electrodes 320 and 321 exposed toward the hollow part 315 of the vacuum cap to function as the treatment unit, and the RF electrodes may be connected to the power supply connectors 433 and 434 when the vacuum cap is coupled to the first body part 410.

A hole communicating with the vacuum tube 431 may be formed at an upper center of the vacuum cap 310, and a tube connector 311 may be provided at an upper end of the hole so as to be connected to the vacuum tube coupler 432 in an airtight state when the vacuum cap is coupled to the first body part 410.

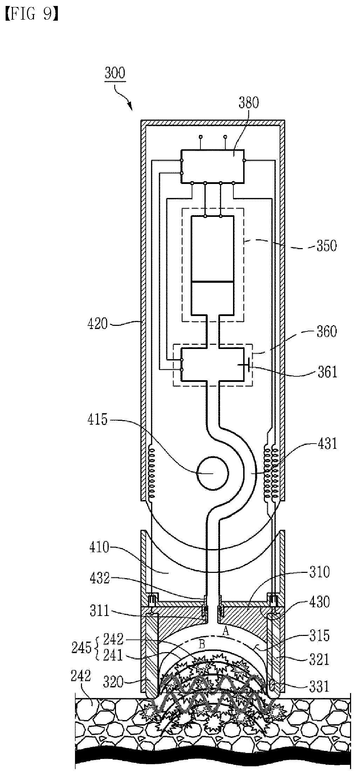

As shown in FIG. 9, the vacuum device 350 including a motor, an exhaust pump, and the like may provide a reference negative pressure into the vacuum cap so that a target site of skin 245 including epidermis 241, fat tissue 242, and the like may be adsorbed toward the hollow part 315 of the vacuum cap so as to make contact with each of the RF electrodes 320 and 321 functioning as the treatment unit.

The reference negative pressure variable device 360 including a pressure variable valve 361 and the like may be configured to serve to change the reference negative pressure applied to the vacuum cap 310 into a pulse form, that is, by a predetermined amplitude in a predetermined cycle for a predetermined time under a condition in which the hollow part 315 of the vacuum cap maintains a negative pressure.

The control unit 380 may perform driving control of the RF electrodes 320 and 321 functioning as the treatment unit, the vacuum device 350, and the reference negative pressure variable device 360.

Although a structure in which the pressure variable valve 361 is provided as a device for providing a fluctuating negative pressure to the reference negative pressure according to the present invention has been described for illustrative purposes, the pressure variable valve 361 may be directly provided in the inside wall of the vacuum cap 310 to vary the pressure in the pulse form based on the reference negative pressure, or an air bag or the like for varying a volume of the hollow part 315 in the pulse form may be installed in a portion of the inside wall of the vacuum cap 310.

The reference negative pressure of the vacuum device 350 for sucking the skin 245 into the vacuum cap 310 may be provided, for example, within a range of -100 mbar to -800 mbar.

In addition, the reference negative pressure variable device 360 may change, for example, the pulse form by ±50 mbar with respect to the reference negative pressure formed by the vacuum device 350 so that the skin 245 sucked into the vacuum cap 310 may be repeatedly contracted and relaxed in a vertical direction.

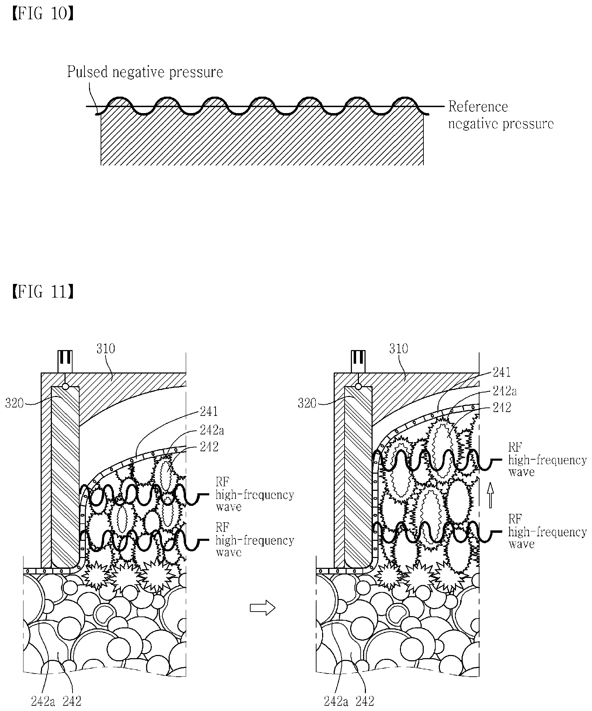

As shown in FIG. 10, the reference negative pressure variable device 360 may form a pulsed negative pressure based on the reference negative pressure, and an amplitude and a wavelength of the pulse may be appropriately controlled by the reference negative pressure variable device 360.

For example, when a positive pressure of 50 mbar is applied into the hollow part 315 by using the pressure variable valve 361 provided in the reference negative pressure variable device 360, a vacuum pressure in the hollow part 315 of the vacuum cap may be decreased, so that the skin 245 including the epidermis 241 and the fat tissue 242 may descend from a position A to a position B as shown in FIG. 9.

On the contrary, when a negative pressure of -50 mbar is applied into the hollow part 315 by using the pressure variable valve 361, the vacuum pressure in the hollow part 315 of the vacuum cap may be increased, so that the skin 245 including the epidermis 241 and the fat tissue 242 may ascend from the position B to the position A.

As described above, a predetermined positive pressure or a predetermined negative pressure may be alternately applied into the hollow part 315 by using the pressure variable valve 361, so that the skin may be repeatedly contracted and relaxed in a pulse wave form at a predetermined interval.

The interval at which the skin is contracted and relaxed in the vertical direction based on a top surface of the target site of the skin may be set, for example, within a range of 0.1 mm or more to 5 mm or less, and a period of a variable pulse of the reference negative pressure formed by the pressure variable valve 361 constituting the reference negative pressure variable device 360 may correspond to 0.5 to 50 times per second.

The RF electrodes 320 and 321 constituting the treatment unit may be mounted on both inside walls of the vacuum cap 310 facing each other as positive (+) and negative (-) electrodes, respectively, and, when RF high-frequency waves are generated from a high-frequency wave generation unit (not shown) and applied to the target site of the skin sucked into the hollow part of the vacuum cap, ions of cell molecules constituting the fat tissue may be configured such that an alternating current of the RF electrode allows positive (+) charges to be attracted to the negative electrode and allows negative (-) charges to be attracted to the positive electrode, resulting in a polarization phenomenon. When Joule heat is intensively generated within fat cell tissue due to the polarization action, fat cells may be decomposed, so that obesity and the like may be treated.

When the skin is repeatedly contracted and relaxed in the vertical direction within the hollow part 315 by using the reference negative pressure variable device 360 according to the present invention, as shown in FIG. 11, a cellulite granular layer 242a that surrounds and protects the fat tissue 242 of the skin may be sufficiently and forcibly relaxed in the vertical direction so as to be broken or disturbed to facilitate transmission of the RF high-frequency waves, so that a decomposition effect of subcutaneous fat tissue may be further promoted as compared with a general treatment device using a vacuum cap, which includes a vibration device or a massage device, according to the related art.

According to the present invention, the handpiece 300 capable of attaching/detaching the vacuum cap and adjusting the angle of the vacuum cap may be provided with a temperature sensor 331 for detecting heat generated during the driving of the RF electrode.

In addition, various cooling devices (not shown) for cooling the skin making contact with the RF electrode, the vacuum cap 310, or the hollow part 315 of the vacuum cap according to a temperature detection signal of the temperature sensor 331 may be additionally provided.

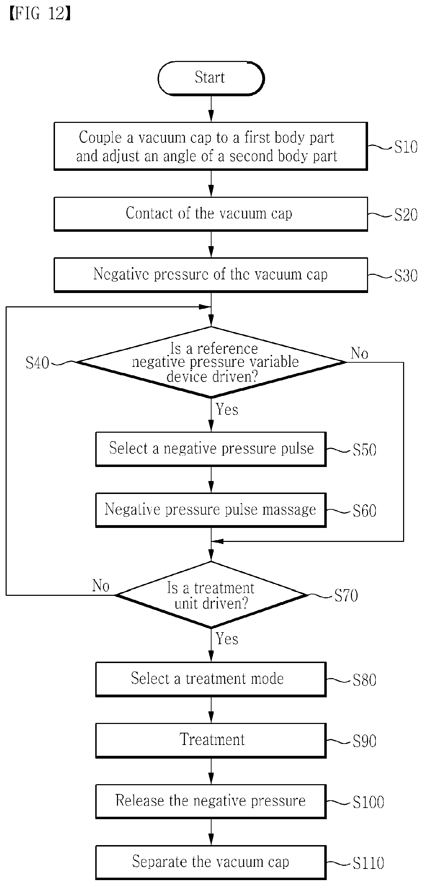

A driving process of the handpiece 300 capable of attaching/detaching the vacuum cap and adjusting the angle of the vacuum cap, which is configured as described above, will be described with reference to FIG. 12.

First, the vacuum cap 310 may be fitted and coupled to the first body part 410, and the angle of the second body part 420 functioning as a handle may be appropriately adjusted (S10).

Thereafter, the target site of the skin 245 may make close contact with an edge of an open portion of the vacuum cap 310 (S20) .

Thereafter, the vacuum device 350 may be driven until the hollow part 315 of the vacuum cap 310 reaches the reference negative pressure (S30) so as to allow the skin 245 to be sucked into the hollow part 315 within the vacuum cap so that the RF electrodes 320 and 321 and a surface of the skin 245 may make contact with each other.

Thereafter, the control unit 380 may determine whether to drive the reference negative pressure variable device 360 according to a control signal of the operator (S40).

When the reference negative pressure variable device 360 is driven, the fluctuating negative pressure for the reference negative pressure may be configured such that a wavelength and an amplitude of a negative pressure pulse are selected within a range of 0.5 to 50 times per second, that is, 0.5 to 50 Hz (S50), and the skin may be massaged because the target site of the skin fluctuates in the vertical direction according to the amplitude of the negative pressure pulse (S60). The amplitude of the negative pressure pulse may be increased in order to increase a vertical movement interval of the skin, and the amplitude of the negative pressure pulse may be decreased in order to decrease the vertical movement interval of the skin. When the wavelength of the negative pressure pulse, that is, a number of fluctuations per second of the fluctuating negative pressure is decreased, a vertical movement speed of the skin may be decreased, and conversely, when the number of fluctuations per second is increased, the vertical movement speed of the skin may be increased.

In addition, the number of fluctuations per second of the fluctuating negative pressure may be controlled by controlling a pulse operation timing of the pressure variable valve 361. In this case, the vacuum device 350 may be configured to automatically operate to maintain the reference negative pressure when the reference negative pressure within the hollow part 315 is out of a predetermined set range due to an adhesion failure occurring in a contact portion between the vacuum cap 310 and the skin.

Thereafter, the control unit 380 may determine whether to drive the RF electrodes 320 and 321 constituting the treatment unit according to a control signal of the operator (S70).

When the treatment unit using electromagnetic waves, such as the RF electrodes, is driven, a treatment mode such as a frequency level, an emission time, and an emission period may be selected (S80), and the RF high-frequency waves may be emitted to the skin according to the selection of the treatment mode so that the fat tissue may be decomposed, or a skin lesion may be treated (S90).

Thereafter, when a treatment is finished, the negative pressure may be released (S100), and the operator may separate the vacuum cap 310 from the first body part 410 of the handpiece (S110).

Meanwhile, when the driving of the reference negative pressure variable device 360 is not selected in the step S40, the step S70 may be performed.

For convenience, it has been described in FIG. 12, that the driving of the reference negative pressure variable device 360 is determined first, and the driving of the RF electrodes 320 and 321 constituting the treatment unit is determined later. The driving of the treatment unit and the reference negative pressure variable device may be operated by selecting one of single driving, simultaneous driving, and alternating driving without being limited to the driving process illustrated above.

A cold and warm heat device, a device using light rays, or the like may be used as the treatment unit instead of the RF electrodes 320 and 321 using the electromagnetic waves.

The handpiece 300 capable of attaching/detaching the vacuum cap and adjusting the angle of the vacuum cap according to the present invention as described above is not limited to the structure and description of the drawings illustrated above, and various modifications can be made without departing from the scope of the claims and objects of the present invention.

### (Description of Reference Numerals)

11, 315 - Hollow part, 12, 13, 320, 321 - RF electrode, 15, 245 - Skin
100, 200 - Treatment device using vacuum cap, 220, 310 - Vacuum cap
242 - Fat tissue, 242a - Cellulite granular layer
300 - Handpiece capable of attaching/detaching vacuum cap and adjusting angle of vacuum cap
311 - Tube connector, 331 - Temperature sensor, 350 - Vacuum device
360 - Reference negative pressure variable device, 361 - Pressure variable valve, 380 - Control unit
410 - First body part, 415 - Rotation shaft, 420 - Second body part
430 - Vacuum cap connection unit, 431 - Vacuum tube, 432 - Vacuum tube coupler
433, 434 - Power supply connector, 435 - Clip

### [Industrial Applicability]

The handpiece capable of attaching/detaching the vacuum cap and adjusting the angle of the vacuum cap according to the present invention may be used in medical devices used for skin care, skin diseases, and obesity treatments.

## Claims

1. A handpiece capable of attaching/detaching a vacuum cap and adjusting an angle of the vacuum cap, the handpiece comprising:
a vacuum cap having an open bottom surface, and formed therein with a hollow part;
a body part to/from which the vacuum cap is attached/detached;
a vacuum device for providing a reference negative pressure to the vacuum cap so that a target site of skin is adsorbed toward the hollow part of the vacuum cap;
a reference negative pressure variable device for varying the reference negative pressure in a pulse form under a condition in which the hollow part of the vacuum cap maintains a negative pressure;
an RF electrode exposed to an inside wall of the vacuum cap so that the adsorbed skin makes contact with an inside the hollow part of the vacuum cap when the target site of the skin is adsorbed toward the hollow part of the vacuum cap; and
a control unit for controlling driving of the vacuum device, the reference negative pressure variable device, and the RF electrode,
wherein the driving of the reference negative pressure variable device and the RF electrode for treating the target site of the skin is controlled by selecting one of single driving, simultaneous driving, and alternating driving, and
the attachment/detachment of the vacuum cap to/from the body part is performed by simultaneous coupling and simultaneous separation of a vacuum cap connection unit including a vacuum tube coupler, a power supply connector, and a clip, which are provided at an end of the body part.

2. The handpiece of claim 1, wherein the body part includes:
a first body part to/from which the vacuum cap is attached/detached; and
a second body part coupled to the first body part with an angle adjustment device, which is configured as a joint member or a rotation shaft, interposed between the first body part and the second body part so that a user bends the second body part to adjust an angle between the first body part and the second body part, and
the vacuum cap connection unit is provided at an end of the first body part.

3. The handpiece of claim 1 or 2, wherein the reference negative pressure variable device is configured to vary the reference negative pressure in the pulse form having a period corresponding to 0.5 to 50 times per second by a pressure variable valve under the condition in which the hollow part of the vacuum cap maintains the negative pressure, and the vacuum device is configured to automatically operate to maintain the reference negative pressure when the reference negative pressure within the hollow part is out of a set range due to an adhesion failure occurring in a contact portion between the vacuum cap and the skin.
